# EUROPÄISCHE PATENTSCHRIFT

(11) **EP 2 361 565 B1**
(45) Veröffentlichungstag und Bekanntmachung des Hinweises auf die Patenterteilung: **21.10.2015**
(21) Anmeldenummer: 11001258.0
(22) Anmeldetag: 16.02.2011
(51) Int. Cl.: A61B 17/29

(54) **Medizinisches Instrument**
Medical instrument
Instrument médical

(30) Priorität: 25.02.2010 DE 102010009259
(43) Veröffentlichungstag der Anmeldung: 31.08.2011
(73) Patentinhaber: Karl Storz GmbH & Co. KG, 78532 Tuttlingen (DE)
(72) Erfinder: Leonhard, Martin, Dr., 78576 Emmingen (DE); Staud, Ralf, 78576 Emmingen (DE)
(74) Vertreter: Hofmeister, Frank

(56) Entgegenhaltungen:
- DE-A1-102007 026 721
- DE-B3-102006 009 559
- US-A1- 2009 131 976

## Beschreibung

Die Erfindung betrifft ein medizinisches Instrument, insbesondere für endoskopische Zwecke, mit einem Schaft, einer am proximalen Ende des Schaftes angeordneten Handhabe, einem am distalen Ende des Schaftes angeordneten Werkzeug mit zwei zwischen einer offenen und einer geschlossenen Stellung verstellbaren Maulteilen sowie mit einem Betätigungselement, über das die Handhabe und das Werkzeug in Wirkverbindung miteinander stehen und wobei jedes Maulteil des Werkzeugs als unter Krafteinwirkung verformbares Maulteil ausgebildet ist, das aus einer inneren Wange und einer äußeren Wange besteht, die am distalen Ende jedes Maulteils miteinander verbunden sind und nach proximal voneinander beabstandet sind, wobei zwischen der inneren Wange und der äußeren Wange mindestens eine Verbindungsstrebe angeordnet ist.

Als Greifinstrumente ausgebildete medizinische Instrumente werden insbesondere in der endoskopischen Chirurgie zum Greifen von Gewebe oder dergleichen biologischem Material verwendet. Die üblicherweise in der Praxis verwendeten medizinischen Greifinstrumente weisen starre Maulteile auf, die keine Flexibilität hinsichtlich des zu ergreifenden Objekts aufweisen. Durch den Kraftschluss zwischen den Maulteilen und dem erfassten Objekt können Punktlasten entstehen, die Schäden am ergriffenen Objekt, beispielsweise einem Blutgefäß, hervorrufen können.

Aus der DE 10 2007 026 721 A1 ist ein formadaptives medizinisches Greifinstrument bekannt, das sich den FinRay-Effekt zunutze macht, um diese nachteiligen Punktlasten zwischen den Maulteilen des Greifinstruments und dem erfassten Objekt zu vermeiden und im Wesentlichen in einen Formschluss zu überführen.

Der FinRay-Effekt beschreibt eine zweischichtige Struktur, die unter Krafteinwirkung eine gerichtete Verformung vollzieht, beispielsweise im Kraftangriffspunkt ausweicht und sich an ihren Enden der Kraftrichtung entgegenbiegt und sich so der Form des die Kraft induzierenden Objekts anpasst.

Die aus der Praxis bekannten adaptiven Greifwerkzeuge weisen jedoch den Nachteil auf, dass die aus einem flachen Bandmaterial gefertigten inneren und äußeren Wangen der Maulteile eine geringe Formstabilität, insbesondere hinsichtlich einer Torsionsbeanspruchung, aufweisen.

Davon ausgehend liegt der Erfindung die **Aufgabe** zugrunde, ein medizinisches Instrument der eingangs genannten Art zu schaffen, dessen Maulteile bei Wahrung der Flexibilität eine erhöhte Formstabilität, insbesondere hinsichtlich der Torsionsbeanspruchung, aufweisen.

Die **Lösung** dieser Aufgabenstellung ist gemäß einer ersten Ausführungsform der Erfindung dadurch gekennzeichnet, dass die innere Wange und die äußere Wange eines jeden Maulteils hinsichtlich der Querschnittsform unterschiedlich ausgestaltet sind, wobei die innere Wange breiter ausgebildet ist als die äußere Wange und die äußere Wange eines jeden Maulteils auf ihrer Außenseite abgerundet oder abgestuft ausgebildet ist, und dass die inneren Wangen der beiden Maulteile am proximalen Ende miteinander verbunden sind, um einen Angriffspunkt für das zum Öffnen und Schliessen der Maulteile dienende Betätigungselement zu bilden.

Die Verbreiterung der inneren Wange vergrößert die Greiffläche dieses Maulteils bei gleichzeitiger Erhöhung des Flächenträgheitsmoments der inneren Wangen. Darüber hinaus bewirkt die abgerundete oder abgestufte Ausgestaltung der Außenseite der äußeren Wange eine Erhöhung der Formstabilität. Insbesondere bei einem endoskopischen Greifinstrument ist diese Anpassung der Außenkontur der äußeren Wangen der Maulteile an die Innenkontur der Trokarhülse, durch die das Greifinstrument ins Operationsgebiet geführt wird, vorteilhaft, da hierdurch der Bauraum bestmöglich ausgenutzt wird, was auch für die Abdichtung der Trokarhülse vorteilhaft ist.

Gemäß einer praktischen Ausführungsform der Erfindung wird vorgeschlagen, dass die innere Wange und die äußere Wange eines jeden Maulteils zusätzlich hinsichtlich der Materialauswahl unterschiedlich ausgestaltet sind.

Die **Lösung** der Aufgabenstellung ist gemäß einer zweiten, alternativen Ausführungsform der Erfindung dadurch gekennzeichnet, dass die innere Wange und die äußere Wange eines jeden Maulteils hinsichtlich der Materialauswahl unterschiedlich ausgestaltet sind, und dass die inneren Wangen der beiden Maulteile am proximalen Ende miteinander verbunden sind, um einen Angriffspunkt für das zum Öffnen und Schliessen der Maulteile dienende Betätigungselement zu bilden.

Durch die hinsichtlich der Querschnittsform und/oder der Materialauswahl unterschiedliche Ausgestaltung der inneren Wange und der äußeren Wange eines jeden Maulteils ist es möglich, das Flächenträgheitsmoment einzelner Wangen zu vergrößern und so bei Beibehaltung der formadaptiven Flexibilität der Maulteile, deren Formstabilität, insbesondere deren Torsionsstabilität, zu erhöhen.

Weiterhin wird mit der Erfindung vorgeschlagen, dass die mindestens eine Verbindungsstrebe zwischen der inneren Wange und der äußeren Wange trapezförmig ausgebildet ist, wenn die äußere Wange schmaler als die innere Wange ausgebildet ist. Diese trapezförmige Ausgestaltung der mindestens einen Verbindungsstrebe bewirkt eine weitere Torsionsbeständigkeit des solchermaßen ausgebildeten Maulteils.

Die Formstabilität der Wangen kann erfindungsgemäß weiterhin dadurch verbessert werden, dass auf der Außenseite der äußeren Wange und/oder der Greiffläche der inneren Wange eines jeden Maulteils eine in Längsrichtung des Maulteils verlaufende Verstärkungsrippe ausgebildet ist.

Um trotz der erhöhten Torsionssteifigkeit der erfindungsgemäß ausgebildeten Maulteile die Flexibilität der Maulteile zu erhöhen, wird mit der Erfindung weiterhin vorgeschlagen, dass in der äußeren Wange und/oder in der inneren Wange eines jeden Maulteils ein fensterartiger Durchbruch ausgebildet ist.

Weiterhin wird mit der Erfindung vorgeschlagen, dass zusätzlich oder alternativ zur Ausbildung der unterschiedlichen Querschnittsformen der beiden Wangen eines jeden Maulteils das Material der inneren Wangen eine größere Steifigkeit aufweist als das Material der äußeren Wangen, um so die Torsionssteifigkeit der Maulteile zu erhöhen.

Die unterschiedlichen Materialien bzw. Materialeigenschaften der inneren und äußeren Wangen lassen sich beispielsweise durch Zweikomponenten-Spritzgussverfahren realisieren, wobei zur Erzielung der unterschiedlichen Steifigkeiten der Wangen beispielsweise faserverstärkte Materialien für die steifere Wange zum Einsatz kommen. Als Material für die Faserverstärkung können beispielsweise Kohlenstoffnanoröhrchen, sogenannte Carbon Nanotubes (CNTs), verwendet werden.

Die Verbindung unterschiedlicher Materialien bzw. Materialeigenschaften hat den Vorteil, dass die Ausgestaltung und das Arbeitsverhalten der Maulteile des Werkzeugs weiter variiert werden können. Beispielsweise können die Maulteile so ausgestaltet werden, dass die inneren Wangen nahezu parallel zueinander schließen oder aber sich zunächst pinzettenartig an den distalen Enden schließen und in der Mitte der Greifflächen einen Hohlraum bilden, je nachdem, welches Greifverhalten des Werkzeugs gewünscht ist.

Bei der Erfindung sind die inneren Wangen der beiden Maulteile am proximalen Ende miteinander verbunden. Diese Verbindungsstelle der beiden inneren Wangen der Maulteile bildet einen geeigneten Angriffspunkt für das Betätigungselement, über das die Maulteile zwischen einer offenen und einer geschlossenen Stellung verstellbar sind. Vorteilhafterweise sind die inneren Wangen der Maulteile und das Betätigungselement als einstückiges Spritzgussteil ausgebildet, so dass eine direkte und spielfreie Verbindung zwischen dem Betätigungselement und dem zu verstellenden Werkzeug besteht.

Um die Fertigung der Maulteile bzw. des gesamten Werkzeugs zu vereinfachen, wird mit der Erfindung weiterhin vorgeschlagen, dass die innere Wange und die äußere Wange eines jeden Maulteils oder die inneren Wangen und die äußeren Wangen beider Maulteile des Werkzeugs als einstückiges Spritzgussteil ausgebildet sind.

Die Verformbarkeit der Maulteile unter Krafteinwirkung kann erfindungsgemäß dadurch verstärkt werden, dass die mindestens eine Verbindungsstrebe über Festkörpergelenke mit der inneren Wange und der äußeren Wange verbunden ist, wodurch eine Relativbewegung der beiden Wangen zueinander erleichtert wird.

Gemäß einer ersten erfindungsgemäßen Ausführungsform zur Ausbildung der Festkörpergelenke wird vorgeschlagen, dass die Festkörpergelenke als Verjüngungen in der Verbindungsstrebe im Übergang zur jeweiligen Wange ausgebildet sind.

Gemäß einer zweiten praktischen Ausführungsform der Erfindung wird vorgeschlagen, dass die Festkörpergelenke als abgerundete Ausnehmungen in der Verbindungsstrebe und/oder in der jeweiligen Wange ausgebildet sind.

Schließlich wird gemäß einer dritten Ausführungsform der Erfindung vorgeschlagen, dass die Festkörpergelenke im Übergang von der Verbindungsstrebe zur jeweiligen Wange mit unterschiedlicher Materialstärke ausgebildet sind.

Weiterhin wird mit der Erfindung vorgeschlagen, dass die inneren Wangen und die äußeren Wangen jedes Maulteils zwischen ihren distal miteinander verbundenen Enden und der distalseitig ersten Verbindungsstrebe flächig miteinander verbunden sind.

Weitere Merkmale und Vorteile der Erfindung ergeben sich anhand der zugehörigen Zeichnungen, in denen vier Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine schematische Seitenansicht eines erfindungsgemäßen medizinischen Instruments;
- Fig. 2: eine vergrößerte Ansicht des Details II gemäß Fi. 1, eine erste Ausführungsform darstellend;
- Fig. 3: einen Schnitt entlang der Linie III-III gemäß Fig. 2;
- Fig. 4: eine Draufsicht auf die Ausführungsform gemäß Fig. 2;
- Fig. 5: eine perspektivische Ansicht des Details V, eine zweite Ausführungsform darstellend;
- Fig. 6: eine Ansicht gemäß Fig. 5, jedoch eine dritte Ausführungsform darstellend und
- Fig. 7: eine Ansicht gemäß Fig. 5, jedoch eine vierte Ausführungsform darstellend.

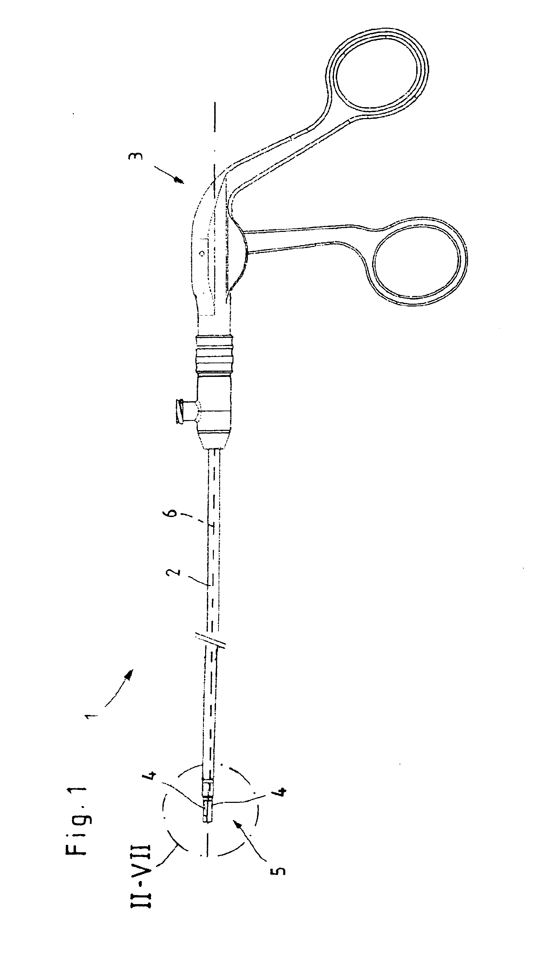

Das in der Abbildung Fig. 1 dargestellte, als Greifinstrument ausgebildete medizinische Instrument 1 besteht im Wesentlichen aus einem Schaft 2, an dessen proximalem Ende eine Handhabe 3 angeordnet ist und an dessen distalem Ende ein aus zwei Maulteilen 4 bestehendes Werkzeug 5 angeordnet ist. Zum Überführen der Maulteile 4 des Werkzeugs 5 von einer offenen Ausgangsstellung in eine geschlossene Stellung stehen die Handhabe 3 und die Maulteile 4 über ein im Schaft 2 gelagertes Betätigungselement 6 in Wirkverbindung miteinander.

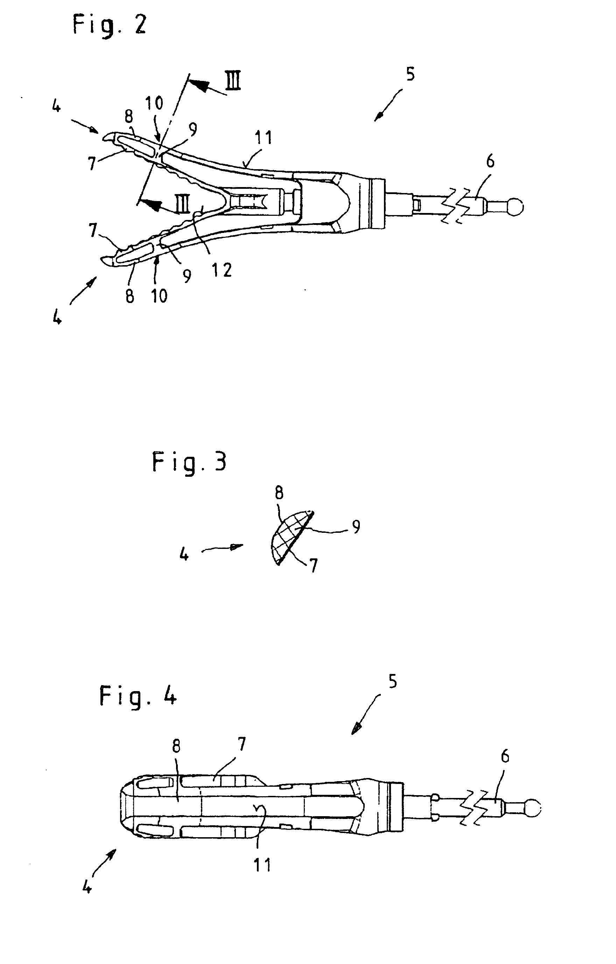

Wie aus Fig. 2 sowie Fig. 5 bis 7 ersichtlich, besteht jedes Maulteil 4 des als Greifwerkzeug 5 ausgebildeten Werkzeugs 5 aus einer inneren Wange 7 und einer äußeren Wange 8, die am distalen Ende eines jeden Maulteils 4 miteinander verbunden sind. Zum proximalen Ende der Maulteile 4 hin sind die innere Wange 7 und die äußere Wange 8 voneinander beabstandet ausgebildet, wobei zwischen der inneren Wange 7 und der äußeren Wange 8 mindestens eine Verbindungsstrebe 9 angeordnet ist.

Als maulteilseitiger Angriffspunkt für das zum Öffnen und Schließen der Maulteile 4 dienende Betätigungselement 6 sind die inneren Wangen 7 der beiden Maulteile 4 an ihrem proximalen Ende miteinander verbunden. Das als Zug-/Druckelemente ausgebildete Betätigungselement 6 greift somit an den inneren Wangen 7 der beiden Maulteile 4 an und bewirkt durch das Ausüben einer Zugkraft auf die inneren Wangen 7 der Maulteile 4 das Schließen der Maulteile 4. Umgekehrt bewirkt die Ausübung einer Druckkraft auf die inneren Wangen 7 der Maulteile 4 das Öffnen der Maulteile 4. Bei den dargestellten Ausführungsformen sind die inneren Wangen 7 direkt an das Betätigungselement 6 angespritzt ausgebildet.

Alternativ zu dieser Ausgestaltung der inneren Wangen 7 sowie des Betätigungselements 6 als einstückiges Spritzgussteil ist es selbstverständlich auch möglich, die inneren Wangen 7 und das Betätigungselement 6 beispielsweise durch Kleben, Nieten, Schrauben oder andere mechanische Kopplungen miteinander zu verbinden.

Die Ausgestaltung der Maulteile 4 mit den beiden voneinander beabstandeten und nur über mindestens eine Verbindungsstrebe 9 miteinander verbundenen Wangen 7 und 8 bewirkt die notwendige Flexibilität der Maulteile 4, die das voranstehend beschriebene Öffnen und Schließen der Maulteile 4 über das Anlegen einer Zug- oder Druckkraft an die inneren Wangen 7 ermöglicht.

Die Flexibilität der Maulteile 4 bis hin zur Ausbildung eines formadaptiven Greifwerkzeugs kann dadurch bewirkt werden, dass die inneren Wangen 7 und äußeren Wangen 8 der Maulteile 4 zur Ausbildung des FinRay-Effekts über ein Fachwerk von Verbindungsstreben 9 miteinander verbunden sind. Der FinRay-Effekt beschreibt eine zweischichtige Struktur (innere Wange 7 und äußere Wange 8), die unter Krafteinwirkung eine gerichtete Verformung vollzieht, beispielsweise im Kraftangriffspunkt ausweicht und sich an ihren Enden der Kraftrichtung entgegenbiegt und sich so der Form des die Kraft induzierenden Objekts anpasst.

Um die Maulteile 4 des Greifwerkzeugs 5 bei Beibehaltung ihrer zur Verformung notwendigen Flexibilität mit einer ausreichend hohe Formstabilität, insbesondere hinsichtlich von Torsionsbeanspruchungen, ausstatten zu können, sind die innere Wange 7 und die äußere Wange 8 eines jeden Maulteils 4 hinsichtlich der Querschnittsform und/oder der Materialauswahl unterschiedlich ausgestaltet, wie dies beispielhaft der Schnittdarstellung gemäß Fig. 3 zu entnehmen ist.

Die Verformbarkeit der Wangen 7 und 8 der Maulteile 4 wird bei den dargestellten ausführungsformen zusätzlich dadurch erhöht, dass die mindestens eine Verbindungsstrebe 9 über Festkörpergelenke 10 mit der inneren Wange 7 und der äußeren Wange 8 verbunden ist, wodurch eine Relativbewegung der beiden Wangen 7 und 8 zueinander erleichtert wird.

In den Abbildungen Fig. 2 und 5 bis 7 sind vier Ausführungsformen zur Ausbildung der Maulteile 4 des Greifwerkzeugs 5 dargestellt, die sich hinsichtlich der Mittel zur Erzeugung der höheren Torsionssteifigkeit der Maulteile 4 und/oder zur Verbesserung der Flexibilität der Maulteile 4 voneinander unterscheiden.

Bei allen dargestellten Maulteilen 4 sind die inneren Wangen 7 breiter ausgebildet als die äußeren Wangen 8, wodurch unter Erhöhung der Torsionssteifigkeit das Flächenträgheitsmoment der inneren Wange 7 vergrößert wird. Zusätzlich zur schmaleren Ausgestaltung der äußeren Wangen 8 sind die äußere Wangen 8 der Maulteile 4 auf ihrer Außenseite 11 abgerundet ausgebildet.

Alternativ zu der dargestellten im Wesentlichen gleichmäßigen Abrundung der Außenseite 11 ist es auch möglich, die Außenseite 11 abgestuft auszubilden.

Insbesondere beim Einsatz des medizinischen Greifinstruments 1 in der endoskopischen Chirurgie ist diese Anpassung der Außenkontur der äußeren Wangen 8 der Maulteile 4 an die Innenkontur der Trokarhülse, durch die das Greifinstrument 1 ins Operationsgebiet geführt wird vorteilhaft, da hierdurch die der Bauraum bestmöglich ausgenutzt wird, was auch für die Abdichtung der Trokarhülse vorteilhaft ist.

In den Abbildungen Fig. 2 bis 4 ist eine erste Ausführungsform zur Ausgestaltung der wie voranstehend beschrieben ausgebildeten Maulteile 4 bzw. inneren Wangen 7 und äußeren Wangen 8 dargestellt. Bei dieser Ausführungsform sind die innere Wange 7 und die äußere Wange 8 über nur eine Verbindungsstrebe 9 miteinander verbunden bzw. von einander beabstandet. Wie aus der Schnittdarstellung gemäß Fig. 3 ersichtlich, ist die Verbindungsstrebe 9 aufgrund der unterschiedlich breit ausgestalteten inneren Wange 7 und äußeren Wange 8 trapezförmig ausgebildet. Diese Trapezform der Verbindungsstrebe 9 erhöht zusätzlich die Torsionsbeständigkeit des Maulteils 4.

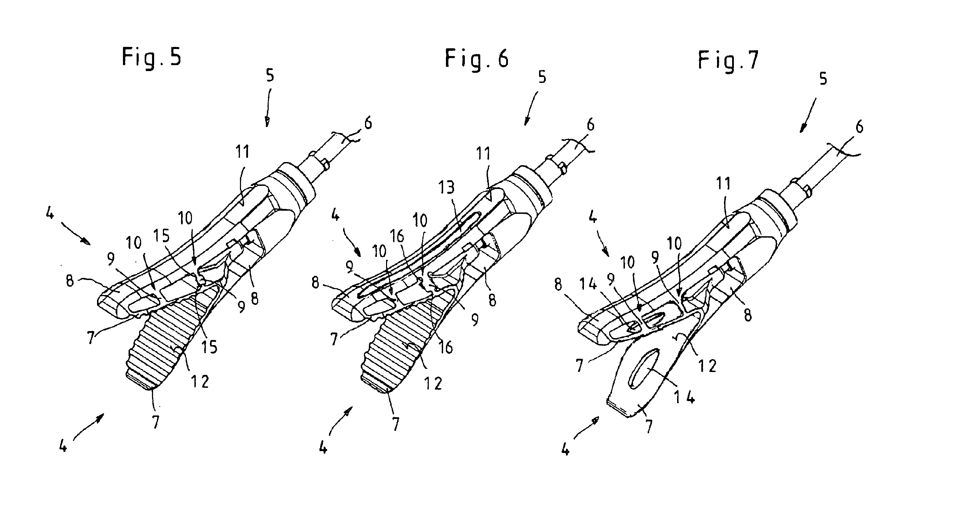

Bei der in Fig. 5 dargestellten zweiten Ausführungsform zur Ausgestaltung der Maulteile 4 bzw. der inneren Wangen 7 und äußeren Wangen 8, sind zwischen der inneren Wange 7 und der äußeren Wange 8 zwei Verbindungsstreben 9 angeordnet. Die dargestellte Anzahl von Verbindungsstreben 9, nämlich nur eine Verbindungsstrebe 9 gemäß Fig. 2 oder zwei Verbindungsstreben 9 gemäß Fig. 5 bis 7, ist nur beispielhaft. Selbstverständlich ist es möglich auch mehr als zwei Verbindungsstreben 9 zu verwenden und diese auch anders anzuordnen, beispielsweise winklig zueinander angestellt. Entscheidend ist, dass die Flexibilität der Maulteile 4 erhalten bleibt.

Weiterhin zeigt die Abbildung Fig. 5, dass die Greifflächen 12 der inneren Wangen 7 bei dieser Ausführungsform als geriffelte Flächen ausgebildet sind. Auch die Ausbildung der Greifflächen 12 in Wellenform ist für entsprechende Anwendungsfälle möglich und sinnvoll. Die geriffelte innere Wange 7 bzw. die innere Wange 7 in Wellenform hat in Verbindung mit Maulteilen 4 aus einem Kunststoff-Material den zusätzlichen Vorteil, dass diese Ausgestaltung wiederum die Flexibilität der inneren Wangen 7 ändert und sich verschiedene Möglichkeiten ergeben, das Verhalten der Maulteile 4 zu bestimmen.

Bei der in Fig. 6 dargestellten dritten Ausführungsform zur Ausgestaltung der Maulteile 4 bzw. der inneren Wangen 7 und äußeren Wangen 8, ist auf den Außenseiten 11 der äußeren Wangen 8 jeweils eine in Längsrichtung der Maulteile 4 verlaufende Verstärkungsrippe 13 angeordnet, durch die sich die Torsionssteifigkeit der Maulteile 4 weiter erhöhen lässt. Alternativ oder auch zusätzlich zur Anordnung einer oder mehrerer Verstärkungsrippen 13 auf den Außenseiten 11 der äußeren Wangen 8 ist es selbstverständlich auch möglich, eine oder mehrere in Längsrichtung der Maulteile 4 verlaufende Verstärkungsrippen 13 auf den Greifflächen 12 der inneren Wangen 7 anzuordnen.

Bei der in Fig. 7 dargestellten vierten Ausführungsform zur Ausgestaltung der Maulteile 4 bzw. der inneren Wangen 7 und äußeren Wangen 8, ist in den inneren Wangen 7 ein fensterartiger Durchbruch 14 ausgebildet, um die Flexibilität der Maulteile 4 zu erhöhen. Alternativ oder auch zusätzlich zur Ausbildung eines oder mehrerer fensterartiger Durchbrüche 14 in den inneren Wangen 7 ist es selbstverständlich auch möglich, einen oder mehrere fensterartige Durchbrüche 14 in den äußeren Wangen 8 auszubilden.

Weiterhin zeigt die Abbildung Fig. 7, dass die Greifflächen 12 der inneren Wangen 7 bei dieser Ausführungsform als ebene glatte Flächen ausgebildet sind

Alternativ und/oder zusätzlich zur Änderung der Querschnittsform der inneren Wangen 7 und äußeren Wangen 8 ist es auch möglich, die Torsionsbeständigkeit durch eine gezielte Materialauswahl zur Ausgestaltung der inneren Wangen 7 und äußeren Wangen 8 zu erzielen, beispielsweise dadurch, dass das Material der inneren Wangen 7 eine größere Steifigkeit aufweist als das Material der äußeren Wangen 8.

Die in den Abbildungen Fig. 5 bis 7 dargestellten Ausführungsformen unterscheiden sich weiterhin durch die Ausgestaltung der Festkörpergelenke 10 voneinander, über die die Verbindungsstreben 9 mit den inneren Wangen 7 und äußeren Wangen 8 verbunden sind.

Bei der in Fig. 5 dargestellten zweiten Ausführungsform sind die Festkörpergelenke 10 der weiter proximalseitig angeordneten Verbindungsstrebe 9 als Verjüngungen 15 in der Verbindungsstrebe 9 im Übergang zur jeweiligen Wange 7 oder 8 ausgebildet.

Dahingegen sind bei der in Fig. 6 dargestellten dritten Ausführungsform die Festkörpergelenke 10 als abgerundete Ausnehmungen 16 in der Verbindungsstrebe 9 und in der jeweiligen Wange 7 oder 8 ausgebildet.

Bei der in Fig. 7 dargestellten vierten Ausführungsform sind die Festkörpergelenke 10 im Übergang von der Verbindungsstrebe 9 zur jeweiligen Wange 7 oder 8 mit unterschiedlicher Materialstärke ausgebildet.

Zusätzlich zu dieser konstruktiven Ausgestaltung der Festkörpergelenke 10 ist es möglich, die Festkörpergelenke 10 so auszubilden, dass sie sich bei Überlastung plastisch verformen ohne jedoch zu brechen, um eine übermäßige Kraftübertragung zu verhindern.

Ein wie voranstehend beschrieben ausgestaltetes medizinisches Greifinstrument 1 zeichnet sich dadurch aus, dass die Maulteile 4 des Greifwerkzeugs 5 aufgrund der besonderen Ausgestaltung der inneren Wangen 7 und äußeren Wangen 8 hinsichtlich der Querschnittsform und/oder der Materialauswahl der Maulteile 4 eine erhöhte Formstabilität, insbesondere hinsichtlich der Torsionsbeständigkeit, aufweisen.

### Bezugszeichenliste

- 1: medizinisches Instrument
- 2: Schaft
- 3: Handhabe
- 4: Maulteil
- 5: Werkzeug/Greifwerkzeug
- 6: Betätigungselement
- 7: innere Wange
- 8: äußere Wange
- 9: Verbindungsstrebe
- 10: Festkörpergelenk
- 11: Außenseite (äußere Wange)
- 12: Greiffläche (innere Wange)
- 13: Verstärkungsrippe
- 14: Durchbruch
- 15: Verjüngung
- 16: abgerundete Ausnehmung

## Patentansprüche

1. Medizinisches Instrument, insbesondere für endoskopische Zwecke, mit einem Schaft (2), einer am proximalen Ende des Schaftes (2) angeordneten Handhabe (3), einem am distalen Ende des Schaftes (2) angeordneten Werkzeug (5) mit zwei zwischen einer offenen und einer geschlossenen Stellung verstellbaren Maulteilen (4) sowie mit einem Betätigungselement (6), über das die Handhabe (3) und das Werkzeug (5) in Wirkverbindung miteinander stehen und wobei jedes Maulteil (4) des Werkzeugs (5) als unter Krafteinwirkung verformbares Maulteil ausgebildet ist, das aus einer inneren Wange (7) und einer äußeren Wange (8) besteht, die am distalen Ende jedes Maulteils (4) miteinander verbunden sind und nach proximal voneinander beabstandet sind, wobei zwischen der inneren Wange (7) und der äußeren Wange (8) mindestens eine Verbindungsstrebe (9) angeordnet ist,
**dadurch gekennzeichnet,**
**dass** die innere Wange (7) und die äußere Wange (8) eines jeden Maulteils (4) hinsichtlich der Querschnittsform unterschiedlich ausgestaltet sind, wobei die innere Wange (7) breiter ausgebildet ist als die äußere Wange (8) und die äußere Wange (8) eines jeden Maulteils auf ihrer Außenseite (11) abgerundet oder abgestuft ausgebildet ist und, dass die inneren Wangen (7) der beiden Maulteile (4) am proximalen Ende miteinander verbunden sind, um einen Angriffspunkt für das zum Öffnen und Schließen der Maulteile (4) dienende Betätigungselement (6) zu bilden.

2. Medizinisches Instrument nach Anspruch 1, **dadurch gekennzeichnet, dass** die innere Wange (7) und die äußere Wange (8) eines jeden Maulteils (4) zusätzlich hinsichtlich der Materialauswahl unterschiedlich ausgestaltet sind.

3. Medizinisches Instrument, insbesondere für endoskopische Zwecke, mit einem Schaft (2), einer am proximalen Ende des Schaftes (2) angeordneten Handhabe (3), einem am distalen Ende des Schaftes (2) angeordneten Werkzeug (5) mit zwei zwischen einer offenen und einer geschlossenen Stellung verstellbaren Maulteilen (4) sowie mit einem Betätigungselement (6), über das die Handhabe (3) und das Werkzeug (5) in Wirkverbindung miteinander stehen und wobei jedes Maulteil (4) des Werkzeugs (5) als unter Krafteinwirkung verformbares Maulteil ausgebildet ist, das aus einer inneren Wange (7) und einer äußeren Wange (8) besteht, die am distalen Ende jedes Maulteils (4) miteinander verbunden sind und nach proximal voneinander beabstandet sind, wobei zwischen der inneren Wange (7) und der äußeren Wange (8) mindestens eine Verbindungsstrebe (9) angeordnet ist,
**dadurch gekennzeichnet,**
**dass** die innere Wange (7) und die äußere Wange (8) eines jeden Maulteils (4) hinsichtlich der Materialauswahl unterschiedlich ausgestaltet sind und, dass die inneren Wangen (7) der beiden Maulteile (4) am proximalen Ende miteinander verbunden sind, um einen Angriffspunkt für das zum Öffnen und Schließen der Maulteile (4) dienende Betätigungselement (6) zu bilden.

4. Medizinisches Instrument nach einem der Ansprüche 1 bis 3, **dadurch gekennzeichnet, dass** die mindestens eine Verbindungsstrebe (9) trapezförmig ausgebildet ist.

5. Medizinisches Instrument nach einem der Ansprüche 1 bis 4, **dadurch gekennzeichnet, dass** auf der Außenseite (11) der äußeren Wange (8) und/oder der Greiffläche (12) der inneren Wange (7) eines jeden Maulteils (4) eine in Längsrichtung des Maulteils verlaufende Verstärkungsrippe (13) ausgebildet ist.

6. Medizinisches Instrument nach einem der Ansprüche 1 bis 4, **dadurch gekennzeichnet, dass** in der äußeren Wange (8) und/oder in der inneren Wange (7) eines jeden Maulteils (4) ein fensterartiger Durchbruch (14) ausgebildet ist.

7. Medizinisches Instrument nach einem der Ansprüche 1 bis 6, **dadurch gekennzeichnet, dass** das Material der inneren Wangen (7) eine größere Steifigkeit aufweist als das Material der äußeren Wangen (8).

8. Medizinisches Instrument nach Anspruch 7, **dadurch gekennzeichnet, dass** die inneren Wangen (7) aus einem faserverstärkten Material bestehen.

9. Medizinisches Instrument nach einem der Ansprüche 1 bis 8, **dadurch gekennzeichnet, dass** die inneren Wangen (7) und das Betätigungselement (6) als einstückiges Spritzgussteil ausgebildet sind.

10. Medizinisches Instrument nach einem der Ansprüche 1 bis 9, **dadurch gekennzeichnet, dass** die innere Wange (7) und die äußere Wange (8) eines jeden Maulteils (4) als einstückiges Spritzgussteil ausgebildet sind.

11. Medizinisches Instrument nach Anspruch 10, **dadurch gekennzeichnet, dass** die inneren Wangen (7) und die äußeren Wangen (8) beider Maulteile (4) des Werkzeugs (5) als einstückiges Spritzgussteil ausgebildet sind.

12. Medizinisches Instrument nach einem der Ansprüche 1 bis 9, **dadurch gekennzeichnet, dass** die mindestens eine Verbindungsstrebe (9) über Festkörpergelenke (10) mit der inneren Wange (7) und der äußeren Wange (8) verbunden ist.

13. Medizinisches Instrument nach Anspruch 12, **dadurch gekennzeichnet, dass** die Festkörpergelenke (10) als Verjüngungen (15) in der Verbindungsstrebe (9) im Übergang zur jeweiligen Wange (7 oder 8) ausgebildet sind.

14. Medizinisches Instrument nach Anspruch 12, **dadurch gekennzeichnet, dass** die Festkörpergelenke (10) als abgerundete Ausnehmungen (16) in der Verbindungsstrebe (9) und/oder in der jeweiligen Wange (7 oder 8) ausgebildet sind.

15. Medizinisches Instrument nach Anspruch 12, **dadurch gekennzeichnet, dass** die Festkörpergelenke (10) im Übergang von der Verbindungsstrebe (9) zur jeweiligen Wange (7 oder 8) mit unterschiedlicher Materialstärke ausgebildet sind.

16. Medizinisches Instrument nach einem der Ansprüche 1 bis 15, **dadurch gekennzeichnet, dass** die inneren Wangen (7) und die äußeren Wangen (8) jedes Maulteils (4) zwischen ihren distal miteinander verbundenen Enden und der distalseitig ersten Verbindungsstrebe (9) flächig miteinander verbunden sind.

## Claims

1. A medical instrument, in particular for endoscopic purposes, having a shaft (2), a handle (3) arranged on the proximal end of the shaft (2), a tool (5) arranged on the distal end of the shaft (2), having two jaw parts (4, which are displaceable between an open position and a closed position, and having an actuating element (6), via which the handle (3) and the tool (5) are operationally connected to one another, and wherein each jaw part (4) of the tool (5) is designed as a jaw part which is deformable under the application of force, and which consists of an inner cheek (7) and an outer cheek (8), which are connected to one another at the distal end of each jaw part (4) and are spaced apart from one another in the proximal direction, wherein at least one connecting strut (9) is arranged between the inner cheek (7) and the outer cheek (8), **characterized in that** the inner cheek (7) and the outer cheek (8) of each jaw part (4) are embodied differently with respect to the cross-sectional shape, wherein the inner cheek (7) is formed wider than the outer cheek (8) and the outer cheek (8) of each jaw part is formed as rounded or stepped on the outer side (11) thereof, and the inner cheeks (7) of the two jaw parts (4) are connected to one another at the proximal end to form an engagement point for the actuating element (6) which is used for opening and closing the jaw parts (4).

2. The medical instrument as claimed in claim 1, **characterized in that** the inner cheek (7) and the outer cheek (8) of each jaw part (4) are additionally embodied differently with respect to the material selection.

3. A medical instrument, in particular for endoscopic purposes, having a shaft (2), a handle (3) arranged on the proximal end of the shaft (2), a tool (5) arranged on the distal end of the shaft (2), having two jaw parts (4) which are displaceable between an open position and a closed position, and having an actuating element (6), via which the handle (3) and the tool (5) are operationally connected to one another, and wherein each jaw part (4) of the tool (5) is designed as a jaw part which is deformable under the application of force, and which consists of an inner cheek (7) and an outer cheek (8), which are connected to one another at the distal end of each jaw part (4) and are spaced apart from one another in the proximal direction, wherein at least one connecting strut (9) is arranged between the inner cheek (7) and the outer cheek (8), **characterized in that** the inner cheek (7) and the outer cheek (8) of each jaw part (4) are embodied differently with respect to the material selection, and the inner cheeks (7) of the two jaw parts (4) are connected to one another at the proximal end to form an engagement point for the actuating element (6) which is used for opening and closing the jaw parts (4).

4. The medical instrument as claimed in any one of claims 1 to 3, **characterized in that** the at least one connecting strut (9) is designed as trapezoidal.

5. The medical instrument as claimed in any one of claims 1 to 4, **characterized in that** a reinforcement rib (13), which extends in the longitudinal direction of the jaw part, is formed on the outer side (11) of the outer cheek (8) and/or the gripping surface (12) of the inner cheek (7) of each jaw part (4).

6. The medical instrument as claimed in any one of claims 1 to 4, **characterized in that** a window-like opening (14) is formed in the outer cheek (8) and/or in the inner cheek (7) of each jaw part (4).

7. The medical instrument as claimed in any one of claims 1 to 6, **characterized in that** the material of the inner cheeks (7) has a greater stiffness than the material of the outer cheeks (8).

8. The medical instrument as claimed in claim 7, **characterized in that** the inner cheeks (7) consist of a fiber-reinforced material.

9. The medical instrument as claimed in any one of claims 1 to 8, **characterized in that** the inner cheeks (7) and the actuating element (6) are formed as a one-piece injection molded part.

10. The medical instrument as claimed in any one of claims 1 to 9, **characterized in that** the inner cheek (7) and the outer cheek (8) of each jaw part (4) are formed as a one-piece injection molded part.

11. The medical instrument as claimed in claim 10, **characterized in that** the inner cheeks (7) and the outer cheeks (8) of both jaw parts (4) of the tool (5) are formed as a one-piece injection molded part.

12. The medical instrument as claimed in any one of claims 1 to 9, **characterized in that** the at least one connecting strut (9) is connected via flexure joints (10) to the inner cheek (7) and the outer cheek (8).

13. The medical instrument as claimed in claim 12, **characterized in that** the flexure joints (10) are formed as tapers (15) in the connecting strut (9) in the transition to the respective cheek (7 or 8).

14. The medical instrument as claimed in claim 12, **characterized in that** the flexure joints (10) are formed as rounded recesses (16) in the connecting strut (9) and/or in the respective cheek (7 or 8).

15. The medical instrument as claimed in claim 12, **characterized in that** the flexure joints (10) are formed having differing material thickness in the transition from the connecting strut (9) to the respective cheek (7 or 8).

16. The medical instrument as claimed in any one of claims 1 to 15, **characterized in that** the inner cheeks (7) and the outer cheeks (8) of each jaw part (4) are connected to one another over a surface between the distal ends thereof, which are connected to one another, and the distal-side first connecting strut (9).

## Revendications

1. Instrument médical, destiné notamment à des fins endoscopiques, comprenant un corps allongé (2), une poignée de manoeuvre (3) agencée à l'extrémité proximale du corps allongé (2), un outil (5) qui est agencé à l'extrémité distale du corps allongé (2) et comporte deux pièces de mâchoire (4) pouvant être déplacées entre une position ouverte et une position fermée, ainsi qu'un élément d'actionnement (6) par l'intermédiaire duquel la poignée de manoeuvre (3) et l'outil (5) sont en liaison d'interaction, instrument
dans lequel chaque pièce de mâchoire (4) de l'outil (5) est configurée sous la forme d'une pièce de mâchoire déformable sous l'action d'une force et est constituée d'une joue intérieure (7) et d'une joue extérieure (8), qui sont reliées l'une à l'autre à l'extrémité distale de chaque pièce de mâchoire (4) et sont écartées l'une de l'autre en direction proximale, et
dans lequel entre la joue intérieure (7) et la joue extérieure (8) est agencée au moins une nervure de liaison (9),
**caractérisé**
**en ce que** la joue intérieure (7) et la joue extérieure (8) de chaque pièce de mâchoire (4) sont de configuration différente quant à leur forme de section transversale, la joue intérieure (7) étant réalisée plus large que la joue extérieure (8), et la joue extérieure (8) de chaque pièce de mâchoire est de configuration arrondie ou étagée sur son côté extérieur (11), et en ce que les joues intérieures (7) des deux pièces de mâchoire (4) sont reliées mutuellement à l'extrémité proximale, pour former un point d'attaque pour l'élément d'actionnement (6) servant à l'ouverture et à la fermeture des pièces de mâchoire (4).

2. Instrument médical selon la revendication 1, **caractérisé en ce que** la joue intérieure (7) et la joue extérieure (8) de chaque pièce de mâchoire (4) sont en plus de configuration différente quant au choix du matériau.

3. Instrument médical, destiné notamment à des fins endoscopiques, comprenant un corps allongé (2), une poignée de manoeuvre (3) agencée à l'extrémité proximale du corps allongé (2), un outil (5) qui est agencé à l'extrémité distale du corps allongé (2) et comporte deux pièces de mâchoire (4) pouvant être déplacées entre une position ouverte et une position fermée, ainsi qu'un élément d'actionnement (6) par l'intermédiaire duquel la poignée de manoeuvre (3) et l'outil (5) sont en liaison d'interaction, instrument
dans lequel chaque pièce de mâchoire (4) de l'outil (5) est configurée sous la forme d'une pièce de mâchoire déformable sous l'action d'une force et est constituée d'une joue intérieure (7) et d'une joue extérieure (8), qui sont reliées l'une à l'autre à l'extrémité distale de chaque pièce de mâchoire (4) et sont écartées l'une de l'autre en direction proximale, et
dans lequel entre la joue intérieure (7) et la joue extérieure (8) est agencée au moins une nervure de liaison (9),
**caractérisé**
**en ce que** la joue intérieure (7) et la joue extérieure (8) de chaque pièce de mâchoire (4) sont de configuration différente quant au choix du matériau, et en ce que les joues intérieures (7) des deux pièces de mâchoire (4) sont reliées mutuellement à l'extrémité proximale, pour former un point d'attaque pour l'élément d'actionnement (6) servant à l'ouverture et à la fermeture des pièces de mâchoire (4).

4. Instrument médical selon l'une des revendications 1 à 3, **caractérisé en ce que** ladite au moins une nervure de liaison (9) est d'une configuration de forme trapézoïdale.

5. Instrument médical selon l'une des revendications 1 à 4, **caractérisé en ce que** sur le côté extérieur (11) de la joue extérieure (8) et/ou sur la surface de prise (12) de la joue intérieure (7) de chaque pièce de mâchoire (4), est formée une nervure de renfort (13) s'étendant dans la direction longitudinale de la pièce de mâchoire.

6. Instrument médical selon l'une des revendications 1 à 4, **caractérisé en ce que** dans la joue extérieure (8) et/ou la joue intérieure (7) de chaque pièce de mâchoire (4), est réalisé un évidement de passage (14) en forme de fenêtre.

7. Instrument médical selon l'une des revendications 1 à 6, **caractérisé en ce que** le matériau des joues intérieures (7) présente une rigidité plus grande que le matériau des joues extérieures (8).

8. Instrument médical selon la revendication 7, **caractérisé en ce que** les joues intérieures (7) sont réalisées en un matériau renforcé de fibres.

9. Instrument médical selon l'une des revendications 1 à 8, **caractérisé en ce que** les joues intérieures (7) et l'élément d'actionnement (6) sont réalisés d'un seul tenant sous la forme d'une pièce moulée par injection.

10. Instrument médical selon l'une des revendications 1 à 9, **caractérisé en ce que** la joue intérieure (7) et la joue extérieure (8) de chaque pièce de mâchoire (4) sont réalisées d'un seul tenant sous la forme d'une pièce moulée par injection.

11. Instrument médical selon la revendication 10, **caractérisé en ce que** les joues intérieures (7) et les joues extérieures (8) des deux pièces de mâchoire (4) de l'outil (5) sont réalisées d'un seul tenant sous la forme d'une pièce moulée par injection.

12. Instrument médical selon l'une des revendications 1 à 9, **caractérisé en ce que** ladite au moins une nervure de liaison (9) est reliée par l'intermédiaire d'articulations monolithiques (10) à la joue intérieure (7) et à la joue extérieure (8).

13. Instrument médical selon la revendication 12, **caractérisé en ce que** les articulations monolithiques (10) sont réalisées en tant que rétrécissements (15) de la nervure de liaison (9) au niveau de la transition à la joue respective (7 ou 8).

14. Instrument médical selon la revendication 12, **caractérisé en ce que** les articulations monolithiques (10) sont réalisées en tant qu'évidements arrondis (16) dans la nervure de liaison (9) et/ou dans la joue respective (7 ou 8).

15. Instrument médical selon la revendication 12, **caractérisé en ce que** les articulations monolithiques (10) sont réalisées avec une épaisseur de matériau différente au niveau de la transition de la nervure de liaison (9) à la joue respective (7 ou 8).

16. Instrument médical selon l'une des revendications 1 à 15, **caractérisé en ce que** les joues intérieures (7) et les joues extérieures (8) de chaque pièce de mâchoire (4) sont reliées mutuellement de manière surfacique entre leurs extrémités reliées mutuellement côté distal et la première nervure de liaison (9) côté distal.
